# EUROPÄISCHE PATENTSCHRIFT

(11) **EP 0 428 831 B1**
(45) Veröffentlichungstag und Bekanntmachung des Hinweises auf die Patenterteilung: **08.06.1994**
(21) Anmeldenummer: 90115303.1
(22) Anmeldetag: 09.08.1990
(51) Int. Cl.: C07D 213/71, C07D 213/61, C07D 213/89

(54) **Verfahren zur Herstellung von Pyridin-3-sulfonsäure**
Process of preparation of pyridine-3-sulfonic acids
Procédé de préparation des acides pyridine-3-sulfoniques

(30) Priorität: 17.11.1989 DE 3938181
(43) Veröffentlichungstag der Anmeldung: 29.05.1991
(73) Patentinhaber: RÜTGERSWERKE AKTIENGESELLSCHAFT, 60326 Frankfurt (DE)
(72) Erfinder: Lösch, Rolf, D-6834 Ketsch (DE); Orth, Windfried, Dr., D-6733 Hassloch/Pfalz (DE); Weiss, Wolfgang, Dr., D-6803 Neckarhausen (DE); Kleffner, Hans Werner, Dr., D-6719 Battenberg (DE)

(56) Entgegenhaltungen:
- US-A- 2 406 972
- US-A- 2 409 806
- CHEMICAL ABSTRACTS, vol. 57, no. 3, 06 August 1962 Columbus, Ohio, USA RusselF.Evans et al.: "The sulfonic acids derived from pyridine and 2,6-lutidine andthe corresponding N-oxides."& J.Org.Chem.Vol.27,p.1329-36,1962 Spalte 1; ref. no. 3405B-G
- CHEMICAL ABSTRACTS, vol. 66, no. 9, 27 Februar 1967 Columbus, Ohio, USA L.Thunus et al.: "Synthesis of 3-and 4-pyridinesulfonic ac &J.Pharm.Belg.vol.21,No.9-10,p.485-90,1966 Seite 3590; Spalte 1; ref. no. 37736H

## Beschreibung

Die Erfindung betrifft ein neues Verfahren zur Herstellung von 3-Pyridinsulfonsäure die in der Galvanotechnik eine gefragte Verbindung ist. Sie dient zur Verbesserung des Abscheideverhaltens von Galvanisierbädern.

Als Zwischenprodukt wird Pyridin-3-sulfonsäure sowohl zur Herstellung von Sulfonamiden und anderen Arzneimitteln als auch für wasserlösliche Reaktivfarbstoffe verwendet.

Bereits 1882 wurde von O. Fischer (Ber. 15, 62 (1882)) die Sulfonierung von Pyridin mit Hilfe von konzentrierter Schwefelsäure bei 300 bis 350 °C beschrieben. Nach 24 stündiger Reaktionszeit konnte Pyridin-3-sulfonsäure in 50 %iger Ausbeute isoliert werden.

Darauf folgende Untersuchungen hatten zum Ziel, die Säure unter milderen Reaktionsbedingungen in einer verkürzten Reaktionszeit in höherer Ausbeute herzustellen.

S. M. MC. Elvain und M. A. Goese fanden heraus, daß die Reaktionstemperatur durch Zugabe von Quecksilbersulfat auf 230 °C gesenkt und die Ausbeute um ca. 20 % gesteigert werden kann (Journal of American Chemical Society 65, 2233 (1943)).

Das derzeit industriell genutzte Verfahren zur Herstellung der Pyridin-3-sulfonsäure beruht im wesentlichen auf den Untersuchungen von Elvain und Goese.

Unter verfahrenstechnischen und ökologischen Gesichtspunkten beurteilt, weist diese Synthesemethode erhebliche Mängel auf.

Hohe Energiekosten entstehen durch die hohe Reaktionstemperatur, die über eine lange Reaktionszeit beibehalten werden muß. Gleichzeitig werden bei der hohen Temperatur aus der konzentrierten Schwefelsäurelösung (Oleum) ätzende SO₃-Dämpfe freigesetzt. Es müssen daher sehr hochwertige, unter diesen aggressiven Bedingunen beständige, Produktionsanlagen für das Verfahren eingesetzt werden.

Das als Katalysator verwendete Quecksilbersulfat muß aufwendig aus dem Produkt entfernt werden, da einerseits in der pharmazeutischen Industrie nur hochreine Produkte eingesetzt werden dürfen, und es andererseits bei der Verwendung in der Galvanotechnik als toxisches Schwermetallsalz auch in den Spuren erhebliche Entsorgungsprobleme mit sich bringt.

Aufgabe der Erfindung ist es, ein umweltfreundliches Verfahren zur Herstellung Schwermetall freier, insbesondere Quecksilber freier, 3-Pyridinsulfonsäure bereitzustellen, das unter milderen Bedingungen bei niedrigerer Temperatur mit verbesserter Ausbeute durchgeführt werden kann.

Die Lösung der Aufgabe erfolgt durch ein Verfahren gemäß den Ansprüchen 1 bis 4.

Als Ausgangsverbindung zur Synthese von 3-Pyridinsulfonsäure bietet sich aus wirtschaftlichen Überlegungen 3-Chlorpyridin an, das als unerwünschtes Nebenprodukt bei der Synthese von 2-Chlorpyridin anfällt, das aber bisher keiner weiteren Verwendung zugeführt werden kann und daher entsorgt werden muß.

Eine Umsetzung des 3-Chlorpyridins in 3-Pyridinsulfonsäure würde demzufolge einerseits das Verfahren zur Herstellung von 2-Chlorpyridin kostengünstiger gestalten, da die Entsorgung entfällt. Andererseits wäre 3-Chlorpyridin zur Herstellung der 3-Pyridinsulfonsäure eine preiswerte Ausgangsverbindung.

Eine Substitution des Chlors in 3-Chlorpyridin durch eine Sulfonsäuregruppe ist jedoch nicht möglich.

Im erfindungsgemäßen Verfahren wird daher in einem ersten Verfahrensschritt 3-Chlorpyridin in das N-Oxid übergeführt wird. Danach erst erfolgt die Sulfonierung nach an sich bekannten Methoden. Das so erhaltene Pyridin-3-sulfonsäure-N-oxid muß nun in einer weiteren Verfahrensstufe, bevorzugt in einer Hydrierreaktion, zur gewünschten Pyridin-3-sulfonsäure reduziert werden.

Um die Darstellung der Pyridin-3-sulfonsäure, mit möglichst geringem Aufwand und mit hoher Ausbeute durchführen zu können, mußte eine Reduktionsmethode gefunden werden, bei der das ungereinigte Pyridin-3-sulfonsäure-N-Oxid direkt umgesetzt wird.

Bekannt sind Reduktionsreaktionen für Pyridin-N-oxide mit elementarem Schwefel, Thioharnstoff, Natriumdithionit, Natriumhydrogensulfit, Natriumsulfit und Phosphortrichlorid. Sie kommen jedoch nicht in Frage, da diese chemischen Reduktionsreaktionen nicht zu einer vollständigen Umsetzung führen, und entstandene Nebenprodukte aufwendig abgetrennt werden müssen.

Eine andere Möglichkeit, den am Stickstoff gebundenen Sauerstoff zu entfernen, bietet sich durch die katalytische Hydrierung an. Hierfür liegen jedoch in der aus der Herstellung des Pyridin-3-sulfonsäure-N-oxids anfallenden Lösung recht ungünstige Ausgangsbedingungen vor, da in der Lösung vorhandene weitere Reaktionspartner, als Katalysatorgifte wirken, und bei der Sulfonierung entstandene Nebenprodukte störend wirken.

Hydrierversuche mit gereinigtem Pyridin-3-sulfonsäure-N-Oxid an Edelmetallkatalysatoren wie z. B. Platin und Palladium zeigen eine zu geringe Selektivität. Der aromatische Kern wird sofort mit hydriert und es entsteht ausschließlich Piperidin-3-sulfonsäure.

Zu den für Hydrierungen im technischen Maßstab einsetzbaren Katalysatoren zählt auch das Raney-Nickel Zur Hydrierung sind meist höhere Temperaturen und Drucke notwendig.

Raney-Nickel wird vorwiegend zur Absättigung aromatischer Systeme und zur Hydrogenolyse organischer Schwefelverbindungen verwendet (vergl. Houben-Weyl, Methoden der organischen Chemie, Bd. 4, Reduktion I, Georg Thieme Verlag, Stuttgart (1980) Seite 21).

Durchgeführt werden diese Reaktionen vorwiegend in alkoholischer Lösung, nachdem das Raney-Nickel mit wasserfreiem Alkohol, z. B. Methanol, gewaschen worden ist.

In heißer alkalischer Lösung werden aromatische Sulfonsäuren in Gegenwart von Raney-Nickel desulfoniert (in: March, Jerry; Advanced Organic Chemistry, Reactions, Mechanisms and Structure, 3rd. Ed., John Wiley & Sons (1985), Seite 509).

Überraschenderweise wurde jedoch gefunden, daß in einem alkalischen Alkohol-Wasser-Gemisch das gereinigte Natriumsalz des Pyridin-3-sulfonsäure-N-oxids unter Erhaltung des aromatischen Kerns mit Raney-Nickel selektiv zu Pyridin-3-sulfonsäure hydriert werden kann. Es ist ein weiterer Vorteil, daß diese Hydrierung unter relativ milden Bedingungen erfolgt.

Darüberhinaus wurde gefunden, daß diese Hydrierung unter etwas härteren Bedingungen auch in basischer wässriger Lösung ohne Zusatz von Alkohol durchführbar ist. Auch unter diesen Bedingungen wird nur die N-O-Bindung hydriert, während die Sulfonsäuregruppe nicht angegriffen wird.

Da die Reinigung des Pyridin-3-sulfonsäure-N-oxids recht aufwendig ist, war es das Ziel, das Verfahren so zu vereinfachen, daß das N-Oxid in ungereinigtem Zustand eingesetzt, d. h. die Reaktionslösung nach der Sulfonierung des 3-Chlor-Pyridin-N-oxids mit dem Hydrierkatalysator versetzt werden kann, so daß die Hydrierung ohne weitere Zwischenstufen durchgeführt werden kann.

Die aus der Herstellung der Sulfonsäure anfallende Lösung bietet für die katalytische Hydrierung recht ungünstige Bedingungen, da vorhandene Sulfitionen und angefallene Nebenprodukte als Katalysatorgifte wirken.

Es wurde gefunden, daß durch die Zugabe von Salzsäure und die dadurch erzielte Entfernung von SO₂ eine Rohlösung entsteht, die zur Hydrierung verwendet werden kann.

Durch Natronlauge wird die Reaktionsflüssigkeit alkalisch gestellt und in Gegenwart von Raney-Nickel hydriert. Bei 100 °C und 7 bar beispielsweise dauert die Wasserstoffaufnahme 6 bis 8 Stunden gegenüber 3 Stunden mit gereinigtem Pyridin-3-sulfonsäure-N-Oxid.

Entgegen aller Erwartungen kann aber auch ohne Abtrennung des Sulfits hydriert werden. Nach der Zugabe von NaOH dauert die Hydrierung unter den gleichen beispielhaften Bedingungen 16 Stunden.

Damit ist es also möglich, die Reinigung des Pyridin-3-sulfonsäure-N-oxids zu umgehen und eine wirtschaftliche Verbindung der Verfahrensschritte zu gewährleisten.

Durch das gefundene Verfahren kann demzufolge schwermetallfreie Pyridin-3-sulfonsäure in technischem Maßstab hergestellt werden.

3-Chlorpyridin wird in an sich bekannter Weise, in organischen Lösemitteln, insbesondere in Eisessig gelöst und mit Hilfe von Oxidationsmitteln bei Temperaturen im Bereich von Raumtemperatur bis etwa 100 °C zu 3-Chlorpyridin-N-oxid oxidiert. Das mit Hilfe von Lösemitteln oder anorganischen oder organischen Extraktionsmitteln oder mittles Destillation mittels üblicher Verfahren abgetrennte 3-Chlorpyridin-N-oxid wird in an sich bekannter Weise mit bekannten Sulfonierungsmitteln, vorzugsweise mit Alkalisulfit, in Gegenwart von Wasser bei erhöhter Temperatur (50 bis 170 °C je nach Aktivität des Sulfonierungsmittels) zu Pyridin-3-sulfonsäure-N-oxid umgesetzt. Es wird eine Umsetzung bis zu 83 % erzielt.

Wird die Sulfonierung mit Alkali-Sulfit durchgeführt, so kann die angefallene Pyridin-3-sulfonsäure-N-oxid haltige Reaktionslösung nun mit einer Säure, bevorzugt Salzsäure versetzt werden, um SO₂ aus der Lösung auszutreiben. Die dabei ausfallenden Salze werden abgesaugt. Die Reaktionslösung wird eingeengt, mit Natronlauge alkalisch gemacht, mit 3 bis 9,5 g Raney-Nickel je 100 g Pyridin-3-sulfonsäure-N-oxid versetzt und bei 80 °C bis 120 °C und 5 bar hydriert. Die Hydrierung wird so lange fortgesetzt, bis keine weitere H₂-Aufnahme mehr feststellbar ist. Dies ist nach etwa 8 bis 12 h erreicht.

Eine zweite Reaktionsvariante besteht darin, SO₂ nicht aus der Reaktionslösung auszutreiben und die aus der Reaktion von Alkalisulfit und 3-Chlorpyridin-N-oxid vorliegende Flüssigkeit direkt mit Natronlauge basisch zu machen und mit Raney-Nickel zu versetzen. So verlängert sich unter sonst gleichen Bedingungen die Hydrierzeit auf 15 bis 20 h. Durch Erhöhen der Katalysatorkonzentration auf 8 bis 15 g Raney-Nickel je 100 g Pyridin-3-sulfonsäure-N-oxid verkürzt sich die Hydrierzeit auf ca. 4 bis 7 h. Nach erfolgter Hydrierung wird die Abtrennung des Reaktionsproduktes durch an sich bekannte Maßnahmen wie Ausfällen, Filtrieren, Extrahieren und Umkristallisieren durchgeführt. Die bevorzugte Reinigungsmethode besteht darin, durch Ansäuern einer Pyridin-3-sulfonsäurelösung noch vorhandenes Sulfit zu entfernen, gebildetes Natriumchlorid durch Einengen und Zugabe von konzentrierter Salzsäure auszufällen und abzufiltrieren, anschließend wird die Mutterlauge weitgehend eingeengt. Mit Toluol als Schleppmittel wird das restliche Wasser abdestilliert. Durch Zugabe von Isopropanol zur abgekühlten Suspension wird das Produkt ausgefällt und das restliche Wasser entfernt. Anschließend wird aus heißem Wasser und/oder niedrigen Alkoholen umkristallisiert. Pyridin-3-sulfonsäure wird in einer Ausbeute von 75 bis 80 % mit einer Reinheit von ca. 90 % und einem SO₂-Aschegehalt von 0,1 % erhalten.

### Beispiele

### Beispiel 1

Zu 113,5 g 3-Chlorpyridin und 250 ml Eisessig werden bei 80 °C 75 g Wasserstoffperoxid (70 Gew.-%) innerhalb von 3 h zugetropft. Nachgerührt wird 5 h bei 80 °C. Nach dem Entfernen des Oxidationsmittels durch Zugabe von Natriumsulfitlösung wird im Wasserstrahlvakuum Essigsäure-Wasser abdestilliert bis die Sumpftemperatur auf 80 °C angestiegen ist. Die Lösung wird abgekühlt und unter Kühlung werden bei 50 °C 157 ml Wasser und 334 g Natronlauge (50 Gew.-%) zugetropft.

Die sich hierbei abscheidende Produktphase wird in 167 ml Toluol aufgenommen. (Bilden sich drei Phasen aus, so muß noch Natronlauge zugegeben werden). Nach der Phasentrennung wird die Laugephase nochmals mit 167 ml Toluol ausgerührt. Die organischen Phasen werden vereinigt und das Toluol in an sich bekannter Weise abdestilliert.
Ausbeute : 98 bis 99 % 3-Chlorpyridin-N-oxid

| | | |
|---|---|---|
| Analyse | Gehalt (Produkt) | 98 bis 99 % |
| | SO₄-Asche | 0,2 bis 0,5 %, |
| | DC | 4 bis 5 Komponenten |

### Beispiel 2

129 g 3-Chlorpyridin-N-oxid aus Beispiel 1, 252 g Natriumsulfit und 700 ml destilliertes Wasser werden 17 h bei 145 °C gerührt. Nach der Reaktion wird das Wasser weitgehend abdestilliert und bei 70 °C mit 500 ml konzentrierter Salzsäure angesäuert. Die Suspension wird 1/2 h gerührt und das Pyridin-3-sulfonsäure-N-oxid in an sich bekannter Weise durch kristallisieren und Trocknen isoliert.

| | |
|---|---|
| Ausbeute an Pyridin-3-sulfonsäure-N-oxid | 76 bis 80 % der Theorie |
| Fp | 247 °C |
| Gehalt | 99,5 % |
| SO₄-Asche | 0,1 % |

### Beispiel 3

51 g (0,29 mol) Pyridin-3-sulfonsäure-N-oxid werden in 200 ml H₂O gelöst. Die Lösung wird mit 25 g Natronlauge (50 Gew.-%) alkalisch gestellt und mit 5 g Raney-Nickel versetzt. Diese Reaktionslösung wird im Autoklaven auf 95 °C erwärmt und bei 7 bar hydriert. Die Hydrierung ist nach 3 Stunden abgeschlossen. Der Katalysator wird abgesaugt und die Mutterlauge eingeengt. Der Rückstand wird in 140 ml Isopropanol und 150 ml konzentrierter Salzsäure gelöst und 4 g Natriumchlorid sowie 4 g Aktivkohle zugegeben. Nach 1 Stunde Rühren wird die Suspension abgesaugt und mit 240 ml Isopropanol versetzt. Pyridin-3-sulfonsäure fällt in Form weißer kristalliner Plättchen aus. Es wird Pyridin-3-sulfonsäure mit einem Chloridgehalt von 0,06 % erhalten.

### Beispiel 4

252 g Natriumsulfit werden unter Ausschluß von Sauerstoff in 700 ml Wasser gelöst. Der Lösung fügt man 129,6 g 3-Chlorpyridin-N-oxid aus Beispiel 1 zu und heizt im Autoklaven auf 145 °C. Das Reaktionsgemisch läßt man 17 h bei 145 °C rühren (Bei der Reaktion stellt sich ein Druck von 4 bis 5 bar ein). Nach Beendigung der Reaktion wird auf 60 °C gekühlt. Zur gekühlten Lösung werden 35 ml Ethanol, 35 g Natronlauge und unter Stickstoff 14 g Raney-Nickel (feucht) gegeben. Die Suspension wird auf 100 bis 110 °C erwärmt, bei dieser Temperatur wird Wasserstoff mit 7 bar aufgedrückt und 6 h hydriert. Nach der Hydrierung wird auf 70 °C abgekühlt und der Katalysator abgesaugt.

Nachgewaschen wird mit Wasser. Von der Flüssigkeit wird die Hälfte des Volumens in Vakuum abdestilliert. Danach gibt man bei 70 °C 250 ml konzentrierte Salzsäure vorsichtig zu. Die Lösung wird bis zur Rührbarkeitsgrenze eingeengt und 500 ml konzentrierte Salzsäure zugegeben. Es wird 1 h bei 40 °C gerührt und dann abgesaugt. Nachgewaschen wird mit Salzsäure. Von der Mutterlauge wird Salzsäure/Wasser im Vakuum weitgehend abdestilliert und Reste an Wasser durch azeotrope Destillation mittels zugegebener 420 ml Toluol entfernt.

Die verbleibende Suspension von Pyridin-3-sulfonsäure in Toluol wird auf 80 °C gekühlt und mit 500 ml Isopropanol versetzt. Danach wird weiter auf ca. 20 °C gekühlt und bei dieser Temperatur 2 h gerührt. Die Suspension wird abfiltriert und die Sulfonsäure mit Isopropanol nachgewaschen. Die erhaltene Sulfonsäure wird in 130 ml destilliertem Wasser heiß gelöst und nach dem Abkühlen auf 70 °C werden 500 ml Ethanol zugegeben. Bei 20 °C wird die Pyridin-3-sulfonsäure abgesaugt. Nachgewaschen wird mit Ethanol.

| | |
|---|---|
| Ausbeute | 75 bis 80 % der Theorie |
| Gehalt (aus Säurezahl) | ca. 99 % |
| SO₄-Asche | 0,1 % |

### Beispiel 5

18,7 kg Natriumbisulfit werden in 55,8 l Wasser gelöst und mit 14,4 kg Natronlauge auf pH 9 bis 9,5 gestellt. Der Lösung fügt man 11,7 kg rohes 3-Chlorpyridin-N-oxid zu und heizt im Autoklaven auf 145 °C. Das Reaktionsgemisch läßt man 17 h bei 145 °C rühren (Bei der Reaktion stellt sich ein Druck von 4 bis 5 bar ein.). Nach Beendigung der Reaktion wird auf 90 °C gekühlt, mit 1 kg Natronlauge (50 Gew.-%) alkalisch gestellt und unter Stickstoff 0,5 kg Raney-Nickel zugegeben. Die Suspension wird auf 100 bis 110 °C erwdämt, bei dieser Temperatur wird Wasserstoff mit 7 bar aufgedrückt und 16 h hydriert. Nach der Hydrierung wird auf 70 °C abgekühlt und der Katalysator mittels eines Druckfilters abgetrennt. Die Pyridin-3-sulfonsäure wird wie in Beispiel 4 isoliert und gereinigt.

### Beispiel 6

252 g Natriumsulfit werden in 700 ml Wasser gelöst. Zu dieser Lösung werden 129,6 g rohes 3-Chlorpyridin-N-oxid hinzugefügt. Unter Stickstoffatmosphäre wird das Reaktionsgemisch auf 145 °C erhitzt und 17 h bei dieser Temperatur gerührt. Anschließend wird auf 60 °C abgekühlt. Es werden 35 ml Ethanol, 35 g Natronlauge (50 %ig) und 14 g Raney-Nickel zugegeben. Die so erhaltene Suspension wird wieder auf 100 °C erwärmt und bei 7 bar 6 h hydriert. Nun wird auf 50 °C abgekühlt und der Katalysator abgesaugt.

Noch in der Reaktionslösung enthaltenes Sulfit wird mit 30 ml Wasserstoffperoxid (70 %) oxidiert. Nun werden im Vakuum etwa 400 ml Wasser vom Reaktionsgemisch abdestilliert und 400 ml Glykol zugesetzt. Weiteres Wasser wird solange abdestilliert bis die Sumpftemperatur auf 100 °C gestiegen ist. Ohne weitere Destillation wird nun durch Verringern des Vakuums die Temperatur auf 130 °C erhöht. Die heiße Suspension wird abgesaugt und der Rückstand mit 100 ml heißem Glykol gewaschen. Vom gesammelten Filtrat wird Glykol abdestilliert, bis ein noch rührbarer Rückstand zurückbleibt, der nach dem Abkühlen mit 500 ml konzentrierter Salzsäure versetzt und eine Stunde bei 35 °C gerührt wird.

Hierbei ausgefallenes Natriumchlorid wird abgesaugt und mit 100 ml konzentrierter Salzsäure gewaschen. Die erhaltenen Filtrate werden vereinigt und bis zur Rührbarkeitsgrenze eingeengt. Anschließend werden bei 80 °C 300 ml Isopropanol zugegeben. Die Suspension wird auf 20 °C abgekühlt und abgesaugt. Der Filterkuchen wird mit 100 ml Isopropanol nachgewaschen.

Die Ausbeute der so erhaltenen Rohsäure beträgt 80 bis 83 %.

Zur weiteren Reinigung wird das Produkt in 260 ml Wasser gelöst. Nach Zugabe von 5 g Aktivkohle (Eponit) wird 30 Minuten bei 80 °C gerührt und anschließend filtriert. Vom Filtrat werden etwa 170 ml Wasser abdestilliert. Es wird auf 70 °C abgekühlt und 350 ml Ethanol hinzugefügt. Nach dem Abkühlen auf 20 °C wird die ausgefallene Pyridin-3-sulfonsäure abgesaugt und mit 100 ml Methanol nachgewaschen.

Die Ausbeute der reinen Pyridin-3-sulfonsäure liegt bei 77 bis 80 % mit einer Reinheit von 99 %.

## Patentansprüche

1. Verfahren zur Herstellung von Pyridin-3-sulfonsäure, **dadurch gekennzeichnet**, daß 3-Chlorpyridin zu 3-Chlorpyridin-N-oxid oxidiert, dieses in wäßriger, alkalischer Lösung zu einem Pyridin-3-sulfonsäure-N-oxid sulfoniert wird und darauffolgend in gleicher Reaktionslösung katalytisch zur Pyridin-3-sulfonsäure reduziert wird.

2. Verfahren nach Anspruch 1, **dadurch gekennzeichnet**, daß 3-Chlorpyridin-N-oxid als Rohprodukt zur Sulfonierung eingesetzt wird.

3. Verfahren nach Anspruch 1, **dadurch gekennzeichnet**, daß Raney-Nickel als Katalysator bei der Reduzierung des Pyridin-3-sulfonsäure-N-oxids verwendet wird.

4. Verfahren nach den Ansprüchen 1 und 2, **dadurch gekennzeichnet**, daß die katalytische Hydrierung von Pyridin-3-sulfonsäure-N-oxid mittels Raney-Nickel in alkalischer wäßriger Lösung in Gegenwart von Sulfitionen und der bei der Sulfonierung gebildeten Nebenprodukten stattfindet.

5. Verfahren nach den Ansprüchen 1 und 2, **dadurch gekennzeichnet**, daß die katalytische Hydrierung von Pyridin-3-sulfonsäure-N-oxid mittel Raney-Nickel, nach dem Entfernen von Sulfit aus der Reaktionslösung durch Säure-Zugabe, in alkalischer wäßriger Lösung, ohne Abtrennung der bei der Sulfonierung gebildeten Nebenprodukte, stattfindet.

## Claims

1. A process for preparing pyridine-3-sulphonic acid, characterized in that 3-chloropyridine is oxidized to form 3-chloropyridine-N-oxide, and the latter is sulphonated in an alkaline, aqueous solution to form pyridine-3-sulphonic acid-N-oxide and is then catalytically reduced in the same reaction solution to form pyridine-3-sulphonic acid.

2. A process according to claim 1, characterized in that 3-chloropyridine-N-oxide is used as a crude product for sulphonation.

3. A process according to claim 1, characterized in that Raney nickel is used as a catalyst in the reduction of the pyridine-3-sulphonic acid-N-oxide.

4. A process according to claim 1 and 2, characterized in that the catalytic hydrogenation of pyridine-3-sulphonic acid-N-oxide takes place by means of Raney nickel in an alkaline aqueous solution in the presence of sulphite ions and the by-products formed during the sulphonation.

5. A process according to claim 1 and 2, characterized in that the catalytic hydrogenation of pyridine-3-sulphonic acid-N-oxide takes place by means of Raney nickel, after the removal of sulphite from the reaction solution by acid addition, in an alkaline aqueous solution, without separation of the by-products formed during the sulphonation.

## Revendications

1. Procédé pour la préparation de l'acide pyridine-3-sulfonique, caractérisé en ce que la 3-chloropyridine est oxydée en 3-chloropyridine-N-oxyde, celle-ci est sulfonée en solution aqueuse, alcaline en acide pyridine-3-sulfonique-N-oxyde et après quoi, dans la même solution de réaction, elle est réduite catalytiquement en acide pyridine-3-sulfonique.

2. Procédé selon la revendication 1, caractérisé en ce que le 3-chloropyridine-N-oxide est mis en oeuvre en tant que produit brut pour la sulfonation.

3. Procédé selon la revendication 3, caractérisé en ce que l'on utilise du nickel de Raney en tant que catalyseur lors de la réduction de l'acide pyridine-3-sulfonique-N-oxide.

4. Procédé selon les revendications 1 et 2, caractérisé en ce que l'hydrogénation catalytique de l'acide pyridine-3-sulfonique-N-oxide s'effectue au moyen du nickel de Raney en solution aqueuse alcaline en présence d'ions sulfites et des sous-produits formés lors de la sulfonation.

5. Procédé selon les revendications 1 et 2, caractérisé en ce que l'hydrogénation catalytique de l'acide pyridine-3-sulfonique-N-oxydes effectue au moyen de nickel de Raney après enlèvement de sulfite de la solution de réaction par addition acide, en solution aqueuse alcaline, sans séparation des sous-produits formés lors de la sulfonation.
